# EUROPEAN PATENT SPECIFICATION

(11) **EP 2 817 049 B1**
(45) Date of publication and mention of the grant of the patent: **04.05.2016**
(21) Application number: 13705000.1
(22) Date of filing: 05.02.2013
(51) Int. Cl.: A61M 15/00

(54) **IMPROVEMENTS RELATING TO MEDICAMENT DELIVERY DEVICES**
VERBESSERUNGEN AN ODER IM ZUSAMMENHANG MIT VORRICHTUNGEN ZUR ARZNEIMITTELABGABE
PERFECTIONNEMENTS SE RAPPORTANT À DES DISPOSITIFS DE DISTRIBUTION DE MÉDICAMENT

(30) Priority: 21.02.2012 GB 201202938
(43) Date of publication of application: 31.12.2014
(73) Proprietor: Innovata Biomed Limited, Edinburgh EH1 2EN (GB)
(72) Inventor: PARKES, Philip Carl, Chippenham Wiltshire SN14 6FH (GB)
(74) Representative: Summersell, Richard John
(86) International application number: PCT/GB2013/050263
(87) International publication number: WO 2013/124618

(56) References cited:
- EP-A2- 0 573 128
- WO-A1-00/64519
- WO-A1-01/31578
- WO-A1-01/41850
- WO-A1-2011/007181
- US-A- 5 575 280
- US-A1- 2003 116 157

## Description

This invention relates to improvements to medicament delivery devices, and in particular to medicament delivery devices in which a unit dose of medicament is transferred from a bulk reservoir by a rotary transfer mechanism. The invention is of particular utility in relation to devices for the administration of medicaments by inhalation, especially dry powder inhalers (DPIs).

The administration of medicaments by inhalation is well-known. A wide variety of medicaments are now administered by that route, for the treatment of a range of respiratory disorders.

The most common form in which such medicaments are formulated for administration by inhalation is as a powder. In the past, many such compositions were formulated as pressurised aerosols, in which the powder medicament was suspended in a liquefied propellant. Due to the adverse environmental effects of the propellants conventionally used, however, there is now increased interest in the use of DPIs. In a DPI, a unit dose of medicament powder, either packaged as such or metered from a bulk reservoir of medicament, is presented to an airway and is then entrained in an airflow passing through the airway. The airflow is most commonly generated by the patient's act of inhalation.

The CLICKHALER® DPI produced by Innovata Biomed Ltd in the UK includes a metering mechanism that is described in EP0539649B. In that device, a metering member is rotatably mounted beneath, and in close abutment with, an opening at the base of a reservoir containing a bulk quantity of powdered medicament. The metering member is formed with a series of depressions that serve as volumetric metering cups. When a cup is in registration with the opening in the reservoir (the filling position), the cup fills with medicament (a unit dose). Rotation of the metering member displaces the cup from that position to a position from where the unit dose can be inhaled (the dispensing position), at the same time bringing the next cup to the filling position. Thus, by indexed rotation of the metering member, the metering cups are successively moved to the filling position, and thereafter to the dispensing position.

WO01/39823 describes another DPI that functions in a similar manner, save that in this device there are two reservoirs containing different medicaments that are to be administered together. Each reservoir is associated with a rotating metering member.

WO2011/007181 describes an actuation mechanism for such a DPI, in which the two metering members are coupled to a common drive wheel that is acted upon by a reciprocating actuator. The actuator is in turn driven by a push-button that is depressed by the user. Each depression of the push-button causes the drive wheel to be rotated, and metering cups on the two metering members to be displaced from their filling positions to their dispensing positions.

DPI devices of the type described above have proven to be very successful. However, it has now surprisingly been found that further improvements in performance, and in particular improvements in the accuracy of the volumetric dosing of the medicament(s) and hence improvements in the uniformity of the dose administered to the user, can be brought about by measures intended to prevent reverse rotation of the metering member(s).

Thus, according to the invention, defined in claim 1, there is provided a medicament delivery device comprising first and second metering members mounted for rotation adjacent to two reservoirs containing bulk quantities of medicament, the metering members being adapted when at a filing position to volumetrically meter desired doses of medicament from the reservoirs and by rotation of the metering members in a first sense to transfer said doses to a dispensing position at which the doses can be dispensed from the device, a drive wheel to which the metering members are operably linked being provided with formations effective to prevent rotation of the metering members from the filling position in the sense opposite to the first sense, the formations effective to prevent rotation of the metering members from the filling position in the sense opposite to the first sense constituting a ratchet-type mechanism, the drive wheel to which the metering members are operably linked on the one hand and an adjacent surface of the device on the other hand being provided with ratchet and pawl formations, wherein the the drive wheel to which the metering members are operably linked is provided with a plurality of pawls that engage, when the metering members are in the filling position, with corresponding ratchet formations to prevent reverse rotation of the metering members.

The medicament delivery device according to the invention is advantageous primarily in that because rotation of the metering members in the reverse sense is prevented, small displacements of the metering members from the filling position, which might otherwise be caused by forces acting on the metering members as a result of operation of the device (e.g. the return of an actuator to its rest position), are eliminated. This may improve the precision of the volumetric dosing of medicament from the reservoirs and so improve the uniformity of the dose received by the user of the device.

The medicament delivery device may be an inhaler, and in particular may be a DPI. However, the invention may find utility in other forms of medicament delivery device, e.g. nasal spray devices and the like.

The device may be used to dispense unit doses of two or more medicaments from the separate reservoirs within the device, those unit doses then being administered to the patient simultaneously.

In the device according to the invention, operation of the device is brought about by means of a push-button or the like, which acts upon an actuator that undergoes a reciprocating motion. By "reciprocating motion" is meant displacement in one direction along a path and then return in the opposite direction along the same path. The path may be linear or have some other suitable form, e.g. the path may be arcuate or radiussed.

The actuator acts upon the drive wheel that is operably linked to the metering members. The device contains two reservoirs of medicament containing different medicaments that are to be administered together. Each reservoir is associated with a separate metering member, and the metering members are coupled to the drive wheel such that rotation of the drive wheel causes rotation of the metering members.

The device contains two reservoirs of medicament, with first and second metering members, and the drive wheel is mounted between the first and second metering members for rotation about a common axis. The first and second metering members may be coupled to the wheel in the manner described in WO2011/007181, i.e. by being provided with sockets that receive the respective ends of a compression spring, the sockets having extensions that project into an axial bushing of the wheel, which bushing in turn is provided on its internal surface with formations that engage the extensions to cause the metering members to rotate when the wheel is rotated.

Rotation of the wheel is brought about by engagement of the actuator with suitable formations provided on the wheel, which take the form of a plurality of lugs that are equiangularly spaced around the wheel. In such an arrangement, the tip of the actuator may lie, in its rest position, adjacent to one such lug, so that downward movement of the actuator drives the lug downwards, and causes the wheel to rotate. Typically, there will be six or eight such lugs, in which case the length of the stroke of the actuator is chosen such that it causes the wheel to rotate through 60° or 45° respectively. The actuator then returns to its rest position, at which point it bears against the next lug on the wheel. The leading surface of the lug may be ramped in order to facilitate return of the actuator to its rest position, the tip of the actuator riding over the following lug on its return stroke.

The drive wheel is provided with a plurality of pawls at its periphery. The number of pawls is most commonly two or four, particularly two. The plurality of pawls are equiangularly spaced. The pawls are preferably resilient and extend slightly from the circumference of the wheel. A number of ratchet formations are formed in a surface of the device adjacent to the periphery of the wheel. Usually, the ratchet formations correspond in number to the number of indexed movements of the metering member that are required to cause one full rotation. So, for instance, if the metering member is formed with eight metering cups having an angular separation of 45° and hence eight actuations of the device are required to cause one full rotation of the metering member, there will be eight ratchet formations. The arrangement is such that whenever the metering member is rotated such that a metering cup occupies a filling position, the pawls engage with a corresponding number of ratchet formations to prevent reverse rotation of the metering member.

As the drive wheel is rotated, the resilience of the pawls allows them to deflect inwardly and so permit rotation. When the wheel has been rotated sufficiently that the next metering cup comes into registration with the opening at the base of its associated reservoir (the filling position), the pawls engage with ratchet formations.

The drive wheel rotates within a generally circular recess formed in a support component that holds the metering member. The ratchet formations are formed in the wall of the circular recess, which is spaced from the edge of the wheel sufficiently to permit rotation in the first sense but is sufficiently close to the wheel to permit engagement of the pawl(s) with the ratchet formations whenever a metering cup is moved into the filling position.

The medicament delivery device is preferably an inhaler, and more preferably a DPI, such as the combination therapy DPI described in WO01/39823.

A variety of medicaments may be administered using the inhaler of the invention.

Such medicaments are generally suitable for the treatment of asthma, COPD and respiratory infections. Such medicaments include, but are not limited to β₂- agonists, eg fenoterol, formoterol, pirbuterol, reproterol, rimiterol, salbutamol, salmeterol and terbutaline; non-selective beta-stimulants such as isoprenaline; xanthine bronchodilators, eg theophylline, aminophylline and choline theophyllinate; anticholinergics, eg ipratropium bromide, oxitropium and tiotropium; mast cell stabilisers, eg sodium cromoglycate and ketotifen; bronchial anti- inflammatory agents, eg nedocromil sodium; and steroids, eg beclomethasone, fluticasone, budesonide, flunisolide, triamcinolone, mometasone and ciclesonide; and/or salts or derivatives thereof.

Specific combinations of medicaments which may be mentioned include combinations of steroids and β₂-agonists. Examples of such combinations are beclomethasone dipropionate and formoterol; beclomethasone dipropionate and salmeterol; fluticasone and formoterol; fluticasone and salmeterol; budesonide and formoterol; budesonide and salmeterol; flunisolide and formoterol; flunisolide and salmeterol; ciclesonide and salmeterol; ciclesonide and formoterol; mometasone and salmeterol; and mometasone and formoterol.

Further medicaments which may be mentioned include systemically active materials, such as proteinaceous compounds and/or macromolecules, for example hormones and mediators, such as insulin, human growth hormone, leuprolide and alpha interferon, growth factors, anticoagulants, immunomodulators, cytokines and nucleic acids.

One embodiment of the invention will now be described in greater detail, by way of illustration only, with reference to the accompanying drawings, in which:
Figure 1 is a perspective view of a dry powder inhaler device according to the invention;
Figure 2 is a simplified sectional view of the metering mechanism of the dry powder inhaler of the present invention;
Figure 3 is a perspective view of a metering member forming part of the dry powder inhaler of the invention;
Figure 4 is a perspective view of a wheel that engages a pair of metering members of the form shown in Figure 3 in the dry powder inhaler of the invention;
Figure 5 shows the wheel of Figure 4 engaged with the metering member of Figure 3;
Figure 6 shows the assembly of wheel and metering member of Figure 5 located within a support member that incorporates a medicament reservoir;
Figure 7 is a side elevation of an actuator that, in use, acts upon the wheel of Figure 4; and
Figure 8 is a side view of the wheel of Figure 4, showing the manner in which the wheel is engaged by the actuator of Figure 7.

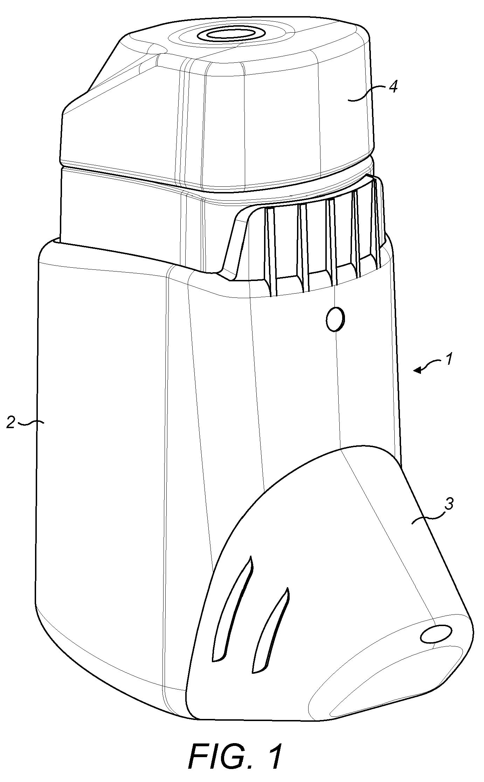

Referring first to Figure 1, a dry powder inhaler is generally designated 1 and comprises a body 2 with an integral mouthpiece that is covered by a removable cap 3. The upper (as viewed in Figure 1) part of the inhaler 1 comprises a depressible push-button 4.

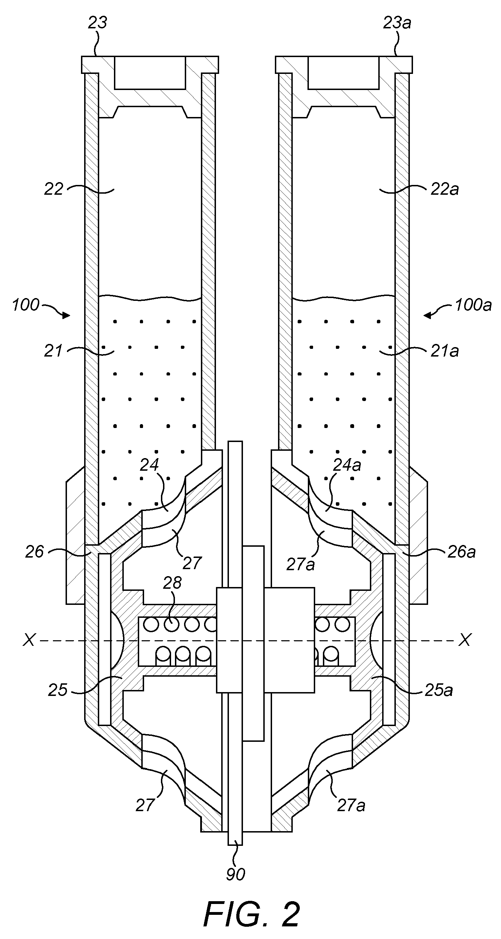

The inhaler 1 is for the simultaneous administration of unit doses of two different powdered medicaments, and includes a metering mechanism that is broadly as described in EP1233805B and is shown in somewhat simplified form in Figure 2.

Referring to Figure 2, the inhaler 1 includes first and second reservoirs 22, 22a that contain bulk quantities of the two medicaments 21, 21a. The reservoirs 22, 22a are arranged side by side and, in normal use of the inhaler 1, substantially vertically. The upper ends of the reservoirs 22, 22a are closed by caps 23,23a.

The reservoir 22 has an opening 24 in its base, from which powdered medicament 21 is discharged from the reservoir 22 under the influence of gravity. A frustoconical metering member 25 is positioned beneath the reservoir 22, such that the surface of the metering member 25 closely abuts the underside of the reservoir 22 so as to close off the opening 24. The reservoir 22 is formed integrally with a frustoconical recess 26 within which the metering member 25 is received. The metering member 25 has a number of depressions 27 formed in its surface. Two such depressions 27 are evident in Figure 2. The depressions 27 serve as metering cups by which a dose of medicament 21 is volumetrically dispensed from the reservoir 22. When a depression 27 is in registration with the opening 24, the depression 27 fills with a dose of powdered medicament 21. Rotation of the metering member 25 then transports that dose to a position from which it can be inhaled by the patient, and at the same time brings another depression 27 into registration with the opening 24.

The other reservoir 22a and associated components are identical in all material respects to those described in the preceding paragraph, save that they are disposed in an arrangement that is the mirror image of that depicted for the first reservoir 22. Components associated with the second reservoir 22a that correspond to components 24, 25, 26, 27 associated with the first reservoir 22 are denoted 24a, 25a, 26a, 27a.

The two metering members 25, 25a are each coupled to a wheel 90 (as described below), and are capable of rotating about a common axis, indicated in Figure 2 by the broken line X-X.

A compression spring 28 is mounted between the metering members 25, 25a, and urges the metering members 25, 25a outwards, into abutment with the respective supports 26, 26a.

The general manner of operation of the inhaler 1 is that depressions 27, 27a that are in registration with the openings 24, 24a are charged with doses of the respective medicaments 21, 21a. When it is desired to administer these doses, the patient causes the metering members 25, 25a to rotate such that the doses are transported to a position from which they can be inhaled. At the same time, empty depressions 27, 27a are brought into registration with the openings 24,24a. Rotation of the metering members 25, 25a is brought about by a ratchet-type mechanism operated by depression of the push button 4, as described below.

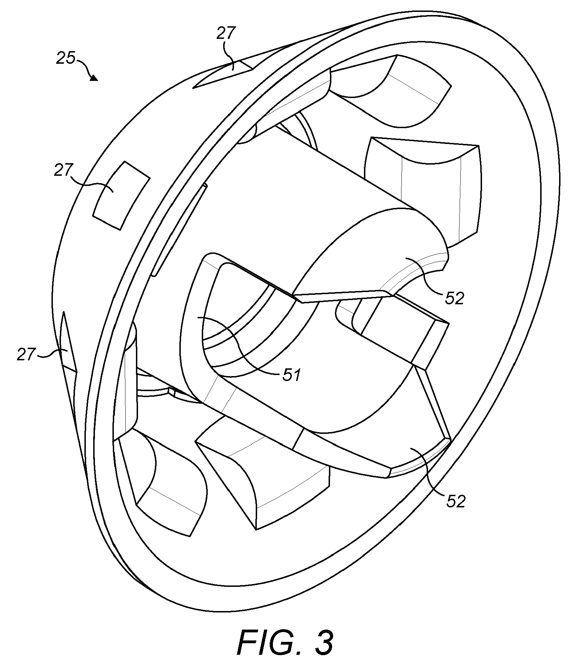

The metering member 25 of the inhaler 1 is shown in detail in Figure 3. As can be seen, the metering member 25 has a generally frustoconical form. The conical part of the outer surface of the metering member 25 is formed with a plurality of depressions (metering cups) 27, that are spaced equiangularly around the metering member 25. In the illustrated embodiment, the metering member 25 has eight such depressions 27.

The underside of the metering member 25 is hollow and is formed with a circular socket 51, from which a pair of diametrically opposed extensions 52 project. The internal diameter of the socket 51 is such that it closely receives the compression spring 28, as described in more detail below. The extensions 52 project beyond the frustoconical part of the metering member 25 and are of arcuate cross-section, being in effect extensions of the circular wall of the socket 51. Each projection 52 has a width that corresponds to a little less than 90° of the circumference of the socket 51. The tips of the projections 52 are tapered.

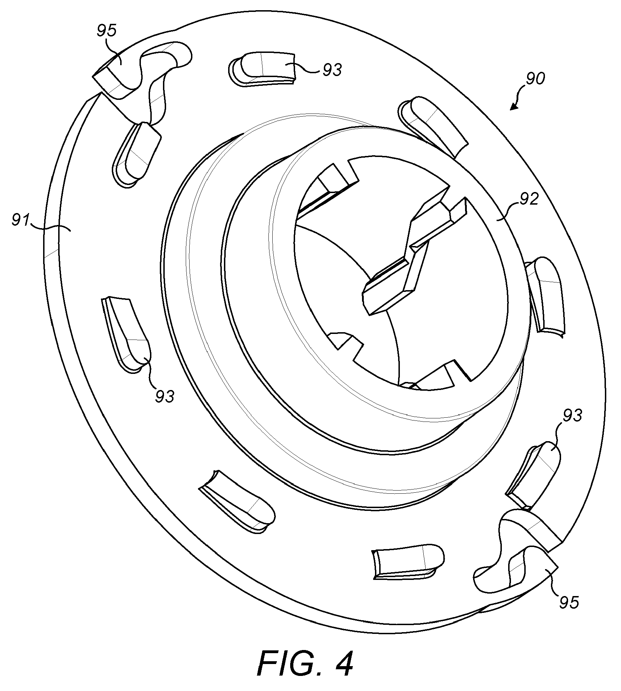

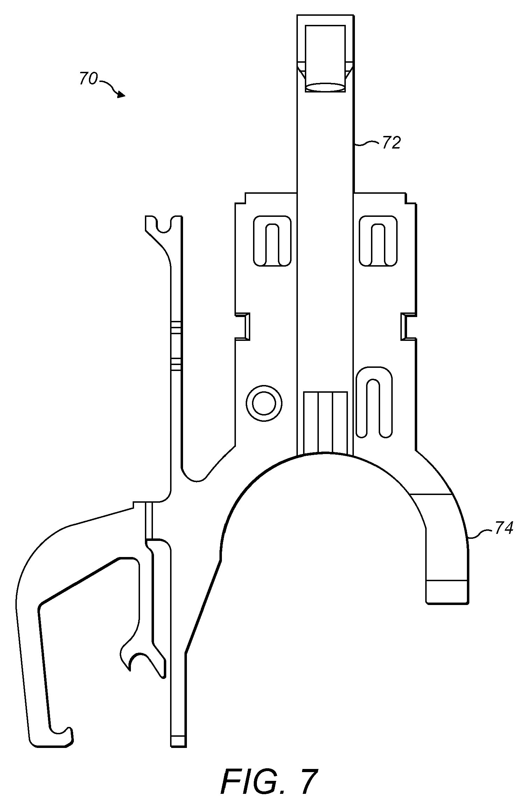

Figure 4 shows a wheel 90 that couples the operation of an actuator (shown in Figure 7 and described more fully below) to the metering members 25, 25a. The wheel 90 comprises a disc 91 with a central cylindrical bushing 92. One face of the disc 91 is formed with a plurality of lugs 93. Each lug 93 has a flat top and a ramped part that extends in the direction of rotation of the wheel 90 (as described below). In the illustrated embodiment, there are eight lugs 93.

As can be seen in Figure 4, the wheel 90 is formed with a pair of pawls 95 that extend a short distance outwardly of the circumference of the remainder of the wheel 90. The wheel 90 is formed by injection moulding in plastics material, with the result that the pawls 95 are resiliently deformable from their rest position (depicted in Figure 4) towards the centre of the wheel 90. To permit flexing movement of the pawls 95, the areas of the disc 91 adjacent to the pawls 95 are cut away with shapes broadly corresponding to that of the pawls 95.

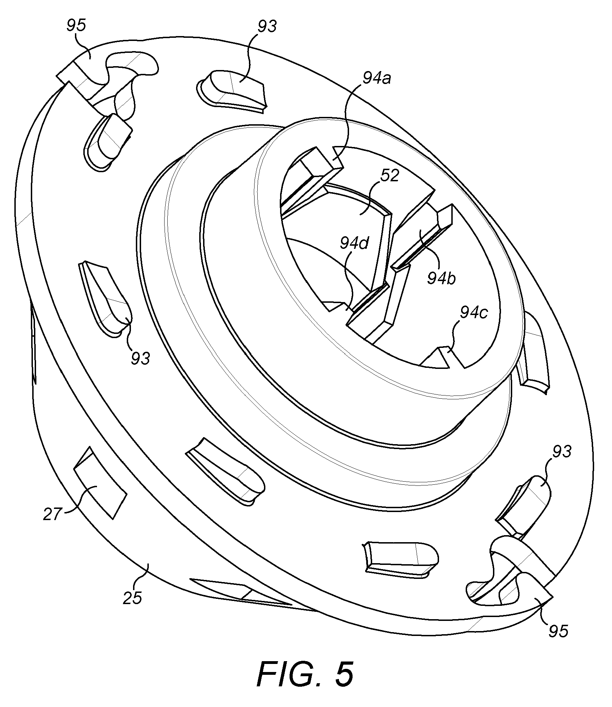

Figure 5 shows the metering member 25 of Figure 3 engaged with the wheel 90. As can be seen in both Figures 4 and 5, the internal surface of the bushing 92 is formed with four splines 94a-d that extend along the full length of the bushing 92, generally parallel to the longitudinal axis of the bushing. The splines 94a-d have a dog-leg or cranked configuration. The effect of this is that, at the end of the bushing 92 that is shown in Figures 4 and 5, the ends of the splines 94a, 94b are separated by an angle of less than 90° and the same is true of the ends of the splines 94c, 94d. The angle between the pairs of splines 94b, 94c and between the splines 94d, 94a, on the other hand, is slightly greater than 90°. At the other end of the bushing 92, it is the pairs of splines 94b, 94c and 94a, 94d that are separated by less than 90°.

The extensions 52 of the metering member 25 are received between the pairs of splines 94 that have a separation of greater than 90° (the width of the extensions 52 is too great for them to be received between the other pairs of splines 94). The tapered tips of the extensions 52, however, are received between the less widely spaced parts of the splines 94 at the other end of the bushing 92. In a similar manner, a second metering member 25a can be engaged with the wheel 90 from the other side, with the extensions 52 of that metering member 25a being received in the spaces between splines 94 that are not occupied by the extensions of the first metering member 25.

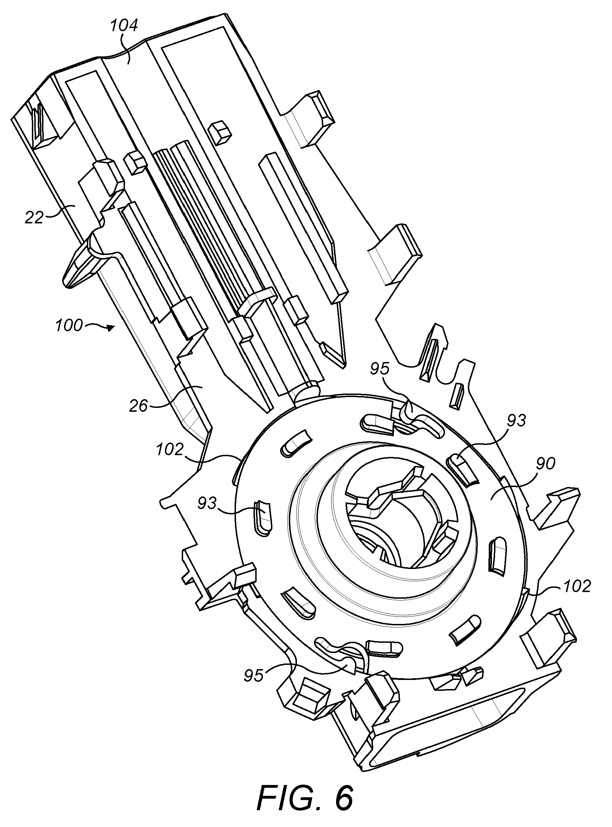

As illustrated in Figure 2, and shown in part but in greater detail in Figure 6, the assembly of metering members 25, 25a and wheel 90 is accommodated between a pair of complementary support members 100,100a, each of which incorporates the respective reservoir 22, 22a and the respective frustoconical recesses 26, 26a. The metering members 25, 25a are closely received within the frustoconical recesses 26, 26a, the metering members 25, 25a being urged into close abutment with those recesses by the compression spring 28.

The ends of the compression spring 28 are received within the circular sockets 51, 51a of the two metering members 25, 25a. Those two sockets 51,51a, together with the extensions 52, 52a, form a substantially complete enclosure for the spring 28, permitting little or no lateral movement of the spring 28. The metering members 25, 25a are effectively coupled to the wheel 90, so that rotation of the wheel 90 causes rotation of both metering members 25, 25a in unison. Nonetheless, because the metering members 25, 25a are not fixed to the wheel 90, movement of the metering members 25, 25a along their axis of rotation is restrained only by the frustoconical recesses 26, 26a in the support members 100,100a within which the metering members 25, 25a are received. The metering members 25, 25a are therefore pressed into close engagement with those recesses 26, 26a by the action of the compression spring 28.

As can be seen in Figure 6, the wheel 90 fits closely within the mouth of the recess 26 in the support member 100. The periphery of the mouth of the recess is formed with eight ratchet formations 102, spaced equiangularly. The pawls 95 engage with a diametrically-opposed pair of such ratchet formations 102. The effect of this is that the wheel 90 is able to rotate in one sense only, namely clockwise (as viewed in Figure 6). Thus, when the wheel 90 is rotated clockwise, the pawls 95 are displaced from the positions they occupy in Figure 6 and are deformed inwardly by the wall of the recess within which the wheel 90 rotates. When the pawls 95 come into registration with the next pair of ratchet formations 102, they engage with those ratchet formations 102. When the pawls 95 are so engaged, rotation of the wheel 90 in the opposite sense (i.e. anti-clockwise in Figure 6) is prevented.

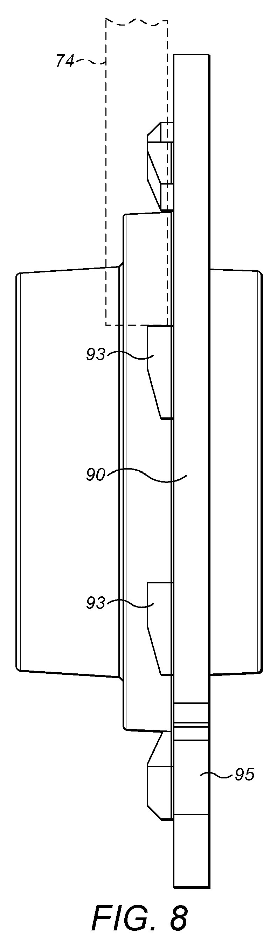

The manner in which the wheel 90 is caused to rotate will now be explained with reference to Figures 7 and 8. An actuator 70 is, in the assembled inhaler 1, captivated between the two support members 100, 100a. The actuator 70 has an upstand 72 that is received in a channel 104 defined by corresponding juxtaposed surfaces of the support members 100, 100a. The actuator 70 is acted upon directly by the push-button 4, such that the actuator 70 is capable of reciprocating motion, being displaced downwardly when the push-button 4 is depressed, and returning to its rest position when the push-button 4 is released. A compression spring (not shown) acts between the lower end of the channel 104 and the actuator 70 to return the latter (and the push button 4) to its rest position.

The lower end of the actuator 70 is formed with a limb 74 that acts on one of the lugs 93 on the wheel 90. In Figure 8, it is the lug 93 at approximately the "three o'clock" position on the wheel 90 that is engaged by the limb 74 (shown in broken lines in Figure 8) on the downstroke of the actuator 70.

The limb 74 presses the lug 93 downwards, causing the wheel 90 to rotate through 45° and displacing the pawls 95 from the positions shown in Figure 6 to the circumferentially next ratchet formation 102. The length of the downstroke of the actuator 70 is sufficient to index the wheel 90 through 45° in that manner, the actuator 70 then returning along its upstroke to its rest position. On the upstroke, the limb 74 rides over the ramped part of the next following lug 93 and comes to rest adjacent to the flat top of that lug 93, so that repeated depression of the push button 4, and hence of the actuator 70, would index the wheel 90 through another 45°.

As noted above, the effect of each actuation is to rotate the wheel 90, and hence both metering members 25, 25a, through 45°. This displaces corresponding metering depressions 27, 27a in the respective metering members 25, 25a from a filling position, at which the metering depressions 27, 27a are gravity fed with medicament from the respective reservoirs 22, 22a, to a dispensing position from which the doses of medicament can be inhaled. At the same time, the next metering depressions 27, 27a are brought into registration with the reservoirs 22, 22a. Because reverse rotation of the wheel 90 (and hence of the metering members 25, 25a) is prevented by engagement of the pawls 95 with the ratchet formations 102, the metering members 25, 25a are held at the filling position. Any tendency for the metering depressions 27, 27a to be slightly displaced from that position, for instance due to frictional or other forces during the upstroke of the actuator 70 as it returns to its rest position, is thereby eliminated. This improves the uniformity of the doses of the two medicaments 21, 21a dispensed from the inhaler 1 during repeated use.

## Claims

1. A medicament delivery device (1) comprising:
two reservoirs (22, 22a) containing bulk quantities of different medicaments (21, 21a) that are to be administered together,
first and second metering members (25, 25a) mounted for rotation adjacent to the reservoirs (22, 22a), wherein each reservoir is associated with a separate metering member, the metering members being adapted when at a filling position to volumetrically meter desired doses of medicament from the reservoirs and by rotation of the metering members in a first sense to transfer said doses to a dispensing position at which the doses can be dispensed from the device, and
a drive wheel (90) mounted between the first (25) and second (25a) metering members for rotation about a common axis (X-X) such that rotation of the drive wheel causes rotation of the metering members, wherein the drive wheel rotates within a generally circular recess (26) formed in a support component (100, 100a) that holds the metering members;
wherein operation of the device is brought about by means of a push-button or the like, which acts upon an actuator that undergoes a reciprocating motion and which actuator acts upon the drive wheel;
wherein rotation of the wheel (90) is brought about by engagement of the actuator (70) with a plurality of lugs (93) that are equiangularly spaced around the wheel (90);
the drive wheel (90) being provided with formations effective to prevent rotation of the metering members from the filling position in the sense opposite to the first sense, these formations constituting a ratchet-type mechanism;
**characterized in that** the drive wheel (90) is provided with a plurality of equiangularly spaced pawls (95) at its periphery that engage, when the metering members (25, 25a) are in the filling position, with corresponding ratchet formations (102) formed in the wall of the circular recess (26) adjacent to the periphery of the wheel and spaced sufficiently from the edge of the wheel to permit rotation in the first sense but sufficiently close to the wheel to permit engagement of the pawls (95) with the ratchet formations (102) whenever a metering cup (27) is moved into the filling position thereby to prevent reverse rotation of the metering members.

2. A medicament delivery device according to Claim 1, which is an inhaler.

3. A medicament delivery device according to Claim 2, which is a dry powder inhaler.

4. A medicament delivery device according to any one of Claims 1 to 3, which is to dispense unit doses of two or more medicaments from separate reservoirs within the device, those unit doses then being administered to the patient simultaneously.

5. A medicament delivery device according to any one of Claims 1 to 4, wherein the wheel is provided with two pawls (95).

6. A medicament delivery device according to any one of Claims 1 to 5, wherein the pawls (95) are resilient and extend a short distance outwardly from the circumference of the wheel (90).

7. A medicament delivery device as claimed in any preceding claim, which is charged with one or more medicaments selected from β₂-agonists, eg fenoterol, formoterol, pirbuterol, reproterol, rimiterol, salbutamol, salmeterol and terbutaline; non-selective beta-stimulants such as isoprenaline; xanthine bronchodilators, eg theophylline, aminophylline and choline theophyllinate; anticholinergics, eg ipratropium bromide, oxitropium and tiotropium; mast cell stabilisers, eg sodium cromoglycate and ketotifen; bronchial anti-inflammatory agents, eg nedocromil sodium; and steroids, eg beclomethasone, fluticasone, budesonide, flunisolide, triamcinolone, mometasone and ciclesonide; and/or salts or derivatives thereof.

8. A medicament delivery device as claimed in any preceding claim, which is charged with a combination of medicaments selected from beclomethasone dipropionate and formoterol; beclomethasone dipropionate and salmeterol; fluticasone and formoterol; fluticasone and salmeterol; budesonide and formoterol; budesonide and salmeterol; flunisolide and formoterol; flunisolide and salmeterol; ciclesonide and salmeterol; ciclesonide and formoterol; mometasone and salmeterol; and mometasone and formoterol.

## Patentansprüche

1. Medikamenten-Verabreichungsvorrichtung (1), umfassend:
zwei Reservoirs (22, 22a), die Bulk-Mengen verschiedener Medikamente (21, 21a) enthalten, die zusammen zu verabreichen sind,
ein erstes und ein zweites Dosierelement (25, 25a), die zur Drehung den Reservoirs (22, 22a) benachbart montiert sind, wobei jedes Reservoir einem getrennten Dosierelement zugeordnet ist, wobei die Dosierelemente geeignet sind, wenn sie in einer Füllposition sind, gewünschte Medikamentdosen aus den Reservoirs volumetrisch zu dosieren und diese Dosen durch Drehung der Dosierelemente in einer ersten Richtung zu einer Verabreichungsposition zu transferieren, in der die Dosen aus der Vorrichtung verabreicht werden können, und
ein Treibrad (90), das zwischen dem ersten (25) und dem zweiten (25a) Dosierelement zur Drehung um eine gemeinsame Achse (X - X) montiert ist, so dass durch eine Drehung des Treibrads eine Drehung der Dosierelemente veranlasst wird, wobei sich das Treibrad in einer allgemein kreisförmigen Aussparung (26) dreht, die in einer Stützkomponente (100, 100a) ausgebildet ist, die die Dosierelemente hält,
wobei eine Betätigung der Vorrichtung mittels einer Taste o. ä. bewirkt wird, die auf einen Aktuator wirkt, der hin- und hergeht und auf das Treibrad wirkt,
wobei eine Drehung des Rads (90) durch Eingriff des Aktuators (70) in eine Vielzahl von Nasen (93) bewirkt wird, die gleichwinklig um das Rad (90) herum beabstandet sind,
wobei das Treibrad (90) mit Ausbildungen versehen ist, die wirksam sind, um eine Drehung der Dosierelemente aus der Füllposition in einer der ersten Richtung entgegengesetzten Richtung zu verhindern, wobei diese Ausbildungen einen ratschenartigen Mechanismus darstellen,
**dadurch gekennzeichnet, dass** das Treibrad (90) an seinem Umfang mit einer Vielzahl von gleichwinklig beabstandeten Klinken (95) versehen ist, die, wenn die Dosierelemente (25, 25a) in der Füllposition sind, die entsprechenden Ratschenausbildungen (102) in Eingriff nehmen, die in der Wand der kreisförmigen Aussparung (26) dem Umfang des Rads benachbart und so ausreichend vom Rand des Rads beabstandet ausgebildet sind, dass sie eine Drehung in der ersten Richtung gestatten, jedoch so ausreichend nahe dem Rad, dass sie immer dann ein Eingreifen der Klinken (95) in die Ratschenausbildungen (102) gestatten, wenn ein Dosierbecher (27) in die Füllposition bewegt wird, so dass dadurch ein Rückwärtsdrehen der Dosierelemente verhindert wird.

2. Medikamenten-Verabreichungsvorrichtung nach Anspruch 1, bei der es sich um einen Inhalator handelt.

3. Medikamenten-Verabreichungsvorrichtung nach Anspruch 2, bei der es sich um einen Trockenpulverinhalator handelt.

4. Medikamenten-Verabreichungsvorrichtung nach einem der Ansprüche 1 bis 3, wobei sie dazu ausgelegt ist, Dosiseinheiten von zwei oder mehr Medikamenten aus getrennten Reservoirs in der Vorrichtung zu verabreichen, wobei diese Dosiseinheiten dann gleichzeitig an den Patienten verabreicht werden.

5. Medikamenten-Verabreichungsvorrichtung nach einem der Ansprüche 1 bis 4, wobei das Rad mit zwei Klinken (95) versehen ist.

6. Medikamenten-Verabreichungsvorrichtung nach einem der Ansprüche 1 bis 5, wobei die Klinken (95) federnd sind und sich vom Umfang des Rads (90) eine kurze Strecke nach außen erstrecken.

7. Medikamenten-Verabreichungsvorrichtung nach einem der vorhergehenden Ansprüche, die mit einem oder mehreren Medikamenten geladen ist, die aus Folgenden ausgewählt sind: β2-Agonisten, z. B. Fenoterol, Formoterol, Pirbuterol, Reproterol, Rimiterol, Salbutamol, Salmeterol und Terbutalin, nichtselektive beta-Stimulanzien wie Isoprenalin, Xanthin-Bronchodilatatoren, z. B. Theophyllin, Aminophyllin und Cholintheophyllinat, Anticholinergika, z. B. Ipratropiumbromid, Oxitropium und Tiotropium, Mastzellenstabilisatoren, z. B. Natrium-Cromoglycat und Ketotifen, bronchiale Entzündungshemmer, z. B. Nedocromil-Natrium, und Steroide, z. B. Beclomethason, Fluticason, Budesonid, Flunisolid, Triamcinolon, Mometason und Ciclesonid und/oder Salze oder Derivate davon.

8. Medikamenten-Verabreichungsvorrichtung nach einem der vorhergehenden Ansprüche, die mit einer Kombination von Medikamenten geladen ist, die aus Folgenden ausgewählt sind: Beclomethasondipropionat und Formoterol, Beclomethasondipropionat und Salmeterol, Fluticason und Formoterol, Fluticason und Salmeterol, Budesonid und Formoterol, Budesonid und Salmeterol, Flunisolid und Formoterol, Flunisolid und Salmeterol, Ciclesonid und Salmeterol, Ciclesonid und Formoterol, Mometason und Salmeterol und Mometason und Formoterol.

## Revendications

1. Dispositif d'administration de médicament (1) comprenant :
deux réservoirs (22, 22a) contenant des quantités en vrac de différents médicaments (21, 21a) destinés à être administrés conjointement,
des premier et second éléments de mesure (25, 25a) installés en vue de leur rotation en position adjacente aux réservoirs (22, 22a), chaque réservoir étant associé à un élément de mesure distinct, les éléments de mesure étant conçus pour mesurer en termes de volume, lorsqu'ils se trouvent au niveau d'une position de remplissage, des doses souhaitées de médicament à partir des réservoirs et, par rotation des éléments de mesure dans un premier sens, transférer lesdites doses à une position de distribution au niveau de laquelle les doses peuvent être distribuées à partir du dispositif, et
une roue d'entraînement (90) installée entre les premier (25) et second (25a) éléments de mesure en vue d'une rotation autour d'un axe commun (X-X), de telle sorte que la rotation de la roue d'entraînement provoque la rotation des éléments de mesure, la roue d'entraînement tournant à l'intérieur d'une cavité globalement circulaire (26) formée dans un composant de support (100, 100a) qui maintient les éléments de mesure ;
le dispositif étant mis en fonctionnement au moyen d'un bouton poussoir ou similaire, qui agit sur un actionneur auquel est imprimé un mouvement alternatif et ledit actionneur agissant sur la roue d'entraînement ;
la roue (90) étant mise en rotation par la coopération de l'actionneur (70) avec une pluralité de protubérances (93) qui sont espacées de façon équiangulaire autour de la roue (90) ;
la roue d'entraînement (90) étant dotée de formations servant à empêcher la rotation des éléments de mesure depuis la position de remplissage dans le sens opposé au premier sens, ces formations constituant un mécanisme à rochet ;
**caractérisé en ce que** la roue d'entraînement (90) est dotée d'une pluralité de cliquets (95) espacés de manière équiangulaire au niveau de sa périphérie qui coopèrent, lorsque les éléments de mesure (25, 25a) se trouvent dans la position de remplissage, avec des formations de rochet (102) correspondantes formées dans la paroi de la cavité circulaire (26) en position adjacente à la périphérie de la roue et suffisamment espacées du bord de la roue pour permettre la rotation dans le premier sens, mais suffisamment proches de la roue pour permettre la coopération des cliquets (95) et des formations de rochet (102) chaque fois qu'un godet de mesure (27) est amené à la position de remplissage de façon à empêcher ainsi la rotation en sens inverse des éléments de mesure.

2. Dispositif d'administration de médicament selon la revendication 1, qui est un inhalateur.

3. Dispositif d'administration de médicament selon la revendication 2, qui est un inhalateur à poudre sèche.

4. Dispositif d'administration de médicament selon l'une quelconque des revendications 1 à 3, qui sert à distribuer des doses unitaires d'au moins deux médicaments à partir de réservoirs séparés à l'intérieur du dispositif, ces doses unitaires étant ensuite administrées au patient de façon simultanée.

5. Dispositif d'administration de médicament selon l'une quelconque des revendications 1 à 4, dans lequel la roue est dotée de deux cliquets (95).

6. Dispositif d'administration de médicament selon l'une quelconque des revendications 1 à 5, dans lequel les cliquets (95) sont élastiques et s'étendent sur une courte distance vers l'extérieur à partir de la circonférence de la roue (90).

7. Dispositif d'administration de médicament selon l'une quelconque des revendications précédentes, dans lequel sont chargés un ou plusieurs médicaments sélectionnés parmi les agonistes β2, par ex. le fénotérol, le formotérol, le pirbutérol, le reprotérol, le rimitérol, le salbutamol, le salmétérol et la terbutaline ; les bêta-stimulants non sélectifs tels que l'isoprénaline ; les bronchodilatateurs de type xanthine, par ex. la théophylline, l'aminophylline et le théophyllinate de choline ; les anticholinergiques, par ex. le bromure d'ipratropium, l'oxitropium et le tiotropium ; les stabilisateurs de membrane, par ex. le cromoglycate de sodium et le kétotifène ; les agents anti-inflammatoires bronchiques, par ex. le nédocromil sodique ; et les stéroïdes, par ex. la béclométhasone, la fluticasone, le budésonide, le flunisolide, la triamcinolone, la mométasone et le ciclésonide ; et/ou les sels ou dérivés de ceux-ci.

8. Dispositif d'administration de médicament selon l'une quelconque des revendications précédentes, dans lequel est chargée une combinaison de médicaments sélectionnée parmi le dipropionate de béclométhasone et le formotérol ; le dipropionate de béclométhasone et le salmétérol ; la fluticasone et le formotérol ; la fluticasone et le salmétérol ; le budésonide et le formotérol ; le budésonide et le salmétérol ; le flunisolide et le formotérol ; le flunisolide et le salmétérol ; le ciclésonide et le salmétérol ; le ciclésonide et le formotérol ; la mométasone et le salmétérol ; et la mométasone et le formotérol.
